(19) Europäisches Patentamt
European Patent Office
Office européen des brevets

(11) EP 1 343 596 B1

(12) **EUROPEAN PATENT SPECIFICATION**

(45) Date of publication and mention of the grant of the patent:
**09.04.2008 Bulletin 2008/15**

(21) Application number: **01270386.4**

(22) Date of filing: **11.12.2001**

(51) Int Cl.:
***B05D 5/08*** *(2006.01)* ***C23C 22/34*** *(2006.01)*
***A61M 15/00*** *(2006.01)* ***C08J 7/04*** *(2006.01)*

(86) International application number:
**PCT/SE2001/002749**

(87) International publication number:
**WO 2002/047829 (20.06.2002 Gazette 2002/25)**

(54) **SURFACE MODIFICATION PROCESS**

OBERFLÄCHENMODIFIZIERUNGSVERFAHREN

PROCEDE DE MODIFICATION DE SURFACE

(84) Designated Contracting States:
**AT BE CH CY DE DK ES FI FR GB GR IE IT LI LU MC NL PT SE TR**

(30) Priority: **13.12.2000 SE 0004610**

(43) Date of publication of application:
**17.09.2003 Bulletin 2003/38**

(73) Proprietor: **AstraZeneca AB**
**151 85 Södertälje (SE)**

(72) Inventors:
- **BREWIS, Derek,**
  **AstraZeneca R & D Charnwood**
  **Loughborough,**
  **Leics. LE11 5RH (GB)**
- **BURNS, Steve,**
  **AstraZeneca R & D Charnwood**
  **Loughborough,**
  **Leics. LE11 5RH (GB)**
- **COLTHORPE, Paul**
  **Nottingham, NG2 7QF (GB)**

(56) References cited:
**EP-A1- 0 565 743** **EP-A1- 1 113 064**
**EP-A2- 0 642 992** **WO-A1-96/32345**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 343 596 B1

## Description

FIELD OF THE INVENTION

The present invention relates to a process for modifying an inside surface of a container and/or a closure and to a container and/or closure modified according to the process of the present invention. In particular, the present invention relates to a process for modifying an inner surface of a canister used for storing a medicament to prevent the medicament from adhering to the canister. Generally the container, closure or canister is one comprising a cap and a metering unit comprising a valve, for delivery of the medicament

BACKGROUND OF THE INVENTION

Adsorption of drugs onto the internal surfaces of aluminium cans, and onto the polymers and metals utilised in valve construction, can be a problem in the development of pressurised metered dose inhaler (pMDI) products. It is known that this adsorption, and consequent reduction in emitted dose, may be to a degree countered by approaches such as the addition of fluorocarbon polymer coating to the internal surface of the aluminium can or, less favourably, the addition of a drug overage. However, there are problems with known approaches. In order to justify the addition of a drug overage it must be shown that adsorbed drug cannot be displaced from the device componentry, and therefore present a risk of overdosing, whereas the addition of a polymer coating to an aluminium can requires the development of a complex coating process and increases the unit cost of the componentry. Also, such coatings are not easily applied to the smaller surfaces of polymer or metal valve components.

Modification of surfaces has traditionally been employed in fields such as textile processing to improve characteristics including repellency, printability and adhesion. The feasibility of modifying the surface of polyethylene terephthalate (PET) using both monomeric and oligomeric silanes with end-capped fluoroalkyl groups has been demonstrated (Kawase et al., J. Adhesion Sci. Technol., Vol. 11, No. 11, pp 1381-1397, 1997). This resulted in a reduction in surface free energy, as measured by contact angles.

Fluorine-containing polymers are known to be useful as protective coatings for various articles. For example, polytetrafluoroethylene (PTFE) has been widely used as a non-stick coating for kitchen utensils, such as frying pans, and tools, such as saws. PTFE and similar fluorine-containing polymers have also found use as hydrophobic protective layers for protecting surfaces against moisture. Published patent applications EP A°0,565,743 and EP A 1,113,064 both disclose the use of fluorosilanes in the formation of hydrophobic and/or non-stick coatings on ceramic surfaces, such as glass. Both of these methods involve the formation of a polymeric layer on the surface, which is useful when producing, for example, cooking utensils.

More recently, Teflon® (polytetrafluoroethylene, PTFE) and perfluoroethylenepropylene have been used to coat the inner surfaces of aluminium canisters intended for use in the storage and administration of .pulmonary medicaments (see EP 0 642 992). Khaladar, Mat. Performance 1994, Vol. 33 part 2, 35-9, discloses fluoropolymer coatings for use as linings, whilst international patent applications WO 96/32150 and WO 96/32345 disclose fluoropolymer coatings for use as linings in the storage and administration of medicaments and in metered dose inhalers respectively. The above coatings are intended to allow alternative improved propellant systems to be used, whilst preventing the contamination of medicaments with, for example, aluminium.

In the process and products described in EP 0 642 992, there is still a requirement that the process used to apply the coatings is improved, to reduce the roughness of the coatings. Also, the coatings sometimes require the addition of an adhesive to the polymer, otherwise the coating does not adhere sufficiently to the surface. Such adhesives may be costly and time consuming to apply, and may be a source of contamination through extractable organic compounds.

It is an aim of the present invention to solve the problems associated with the prior art, in particular with the known processes discussed above. It is therefore an aim of the present invention to provide a simple, inexpensive, chemical process to modify the internal surface of a container, especially the canister and valve surfaces of a pMDI, in order to reduce drug deposition and hence eliminate the need for a drug overage or polymer coat. It is also an aim of the present invention to provide an improved process for modifying an internal surface of a medicine storage container, to provide a surface with improved protective properties and which contains a minimum of extractable organic compounds.

SUMMARY OF THE INVENTION

Accordingly, the present invention provides a method for modifying an internal surface of a container or closure, which method comprises contacting the internal surface with a fluorine-containing silane compound to form a modified surface, wherein the container is suitable for storing a medicament. The silane compounds have formula (I) or (II) as defined in claim 1.

The modified surface is optically transparent, colourless, and chemically stable. The modification can be applied to metallic and polymeric canisters prepared in a commercial manner. A superior modification can be realised by special surface treatment of the container, by specific application of the modifying compound (including the amount and distribution of the silane) and by specific adaptation .of the application equipment and test protocols. It is these aspects that are the subject of this invention.

DETAILED DESCRIPTION OF THE INVENTION

The invention will now be described in further detail by way of example only, with reference to the accompanying drawings, in which:

Figure 1 shows XPS (X-ray Photoelectron Spectroscopy) data for fluorine-containing silane treatment of a number of test substrates, including unmodified polybutylene terephthalate (PBT), PBT modified with 1H,1H,2H,2H-perfluoroooctyltrimethoxysilane (MFS) and PBT modified with 1H,1H,2H,2H-perfluorooctyltriethoxysilane (EFS), unmodified aluminium and aluminium modified with MFS;

Figure 2 shows contact angle data for fluorine-containing silane treatment of a number of test substrates, including unmodified PBT, PBT modified with MFS and PBT modified with EFS, unmodified aluminium and aluminium modified with MFS (mean+SD, n=4);

Figure 3 shows XPS data for a control surface (untreated, degreased aluminium) and aluminium surfaces treated, at either ambient temperature or 130°C, with four differing fluorine-containing silane formulations, A, B, C, and D;

Figure 4 shows contact angle data for a control surface (untreated, degreased aluminium) and aluminium surfaces treated, at either ambient temperature or 130°C, with four differing fluorine-containing silane formulations, A, B, C, and D; and

Figure 5 shows XPS and AES (Auger Electron Spectroscopy) data for a control (an untreated, degreased, aluminium can) and aluminium cans treated at 130°C with fluorine-containing silane formulation C.

The process of the present invention will now be described in more detail. The silane modifying agent is reacted with the surface by contacting the surface to be modified with the silane. The method of contacting the surface with the silane is not especially limited. It may comprise plasma coating, immersion of the surface in a bath comprising the silane, spraying the silane onto the surface, or applying the silane to the surface with an applicator, such as with a brush in the manner of painting. Preferably, however, the surface is contacted with the silane by plasma coating or by immersion. Plasma coating is particularly preferred for plastics surfaces.

Plasma coating has been used in the past for forming a thin-layer polymer coating on surfaces. For example, GB 2,355,252 discloses a 'cold plasma' coating procedure for forming a polymerised silazine, siloxane or alkoxysilane layer on a surface of drug delivery device components. This procedure can be adapted for use in the present invention to modify aluminium or plastics surfaces with fluorine-containing silane monomers.

It is preferred that a cold plasma technique is employed, particularly when coating plastics materials such as PBT and acetal; since plastics can be coated without causing any damage due to heating. This is because in a cold plasma treatment, the temperature of the plasma is at around the ambient temperature. The treatment is carried out in a vacuum, and the surface to be modified is placed within the vacuum, preferably inside an evacuated chamber. The strength of the vacuum is not especially limited, provided that it is sufficient to allow plasma coating to occur, but preferably the vacuum is at a pressure of 0.667 Pa (0.005 Torr) or less. The fluorine-containing silane compound (and a fluorine-containing carbon compound in a special embodiment) is introduced at a controlled rate into the vacuum chamber in which the surface to be modified is situated. A radio frequency signal is applied via an external antenna to form the plasma. It will be clear to the skilled person that the appropriate frequency may be selected, depending upon the particular fluorine-containing silane compound employed. Generally frequencies of the order of 10 MHz are useful in the present invention, although lower or higher frequencies may be employed, depending upon the substance being employed to modify the surface. The power of the RF signal is not especially limited, provided that it is sufficient to 'ignite' the plasma and promote coating. Typically a power of from 50 to 100 W is employed. The plasma is 'ignited' within the chamber and maintained for a selected time at a pre-selected power setting. Once the treatment is complete, the radio frequency is switched off to 'extinguish' the plasma. The chamber is then flushed, and the products retrieved. As a result of the procedure, a thin layer of monomer is attached to the surface to be modified. The layer thickness that can generally be achieved is from about 0.005 to 0.5 $\mu$m

In other coating methods, the silane may be provided in any form appropriate for surface modification to occur.

Generally, and in particular when applied by immersion, the silane is provided as a solution in a solvent composition. The solvent composition is not especially limited, provided that it does not prevent the modification of the surface from taking place. Typically the solvent composition comprises an organic non-polar solvent, preferably having a low boiling point Preferred solvents include fluorocarbons and perfluorocarbons (e.g. Freons®), hydrofluoroalkanes (HFAs), chlorinated hydrocarbons (e.g. trichloroethylene, tetrachloromethane, trichloromethane, and dichloromethane), ethers (e.g. dibutyl ether), ketones (e.g. methylethylketone), and alkanes (e.g. hexane). The most preferred solvents include inert fluorocarbon solvents, such as Freon® 22, Freon® 112 and/or Freon® 113. The solvent composition may optionally also comprise one or more polar components. Without being bound by theory, it is believed that such components help to solubilise the hydrolysed silane intermediate before it reacts with the surface to be modified. Such polar components include polar solvents, such as methyl ethyl ketone (MEK), acetone, ether, alcohols (e.g. methanol or ethanol), acetonitrile and even water. The polar component may be present in larger quantity than the organic non-polar solvent, if desired. The solvent composition may further comprise mild acids, such as mineral and organic acids, if required. Preferred acids include dilute acetic acid, and/or dilute hydrochloric acid. The presence of acids is preferred (especially for modifying metal surfaces) since acids hydrolyse the silane compound, activating it for reaction with the container surface. The solvent composition may optionally comprise still further additives for improving the modification process, such as wetting agents and/or surfactants, where these are believed to be beneficial.

The concentration of the silane in the solvent composition is not especially limited, provided that the modification process is not adversely affected. Preferably, the solvent composition comprises from 0.01-25 vol.% of silane, more preferably from 0.1-5 vol.% of silane.

In some embodiments of the present invention (especially when the solvent composition does not comprise an acid) it is preferred to allow the solvent composition to stand for a time, after it has been prepared. Without being bound by theory, it is believed that this allows some hydrolysis of the silane compound to occur, which promotes attachment to the surface. Generally, it is preferred that the solvent composition is allowed to stand for from 10-60 minutes, most preferably for about 30 minutes.

The time period over which immersion is carried out is not especially limited, provided that the degree of modification is suitable for achieving the desired advantages outlined above. Immersion times may be varied dependent on the type of surface, the type of silane and the solvent system employed. Typically, however, the immersion is carried out for 10 mins or less.

In some aspects of the present invention, the modification step may be repeated one or more times. Repetition of the modification step may help to further improve the degree of modification of the surface, ensuring that the properties of the surface are optimised.

Preferably, before the modification step is carried out, the surface to be modified is prepared for modification. The preparation step has the effect of freeing the surface from unwanted species that may interfere with, or hinder the modification step, or prevent the modification taking place at all. Preparation typically involves cleaning the surface, for example with a cleaning composition comprising a solvent, a detergent and/or a surfactant. Solvents which do not leave a residue are preferred, especially volatile solvents. Preferred solvents include methylethylketone (MEK), acetone, and iso-propyl alcohol (IPA). Preferred detergents and surfactants include sodium lauryl sulphate and the Tweens®. Other preparation steps may also be carried out, in addition to or instead of cleaning, such as contacting the surface with an acid. Acid treatments, as well as having a cleaning function, typically also provide some degree of activation of the surface to be modified, facilitating the modification step. Mineral acids, such as sulphuric acid and nitric acid are preferred, in particular dilute acids, such as aqueous hydrochloric acid and aqueous sulphuric acid. Further preparation steps may include agitation to remove debris, such as ultrasonic agitation.

In the method of the present invention, after the modification step the modified surface is preferably washed. This helps to remove unwanted substances, such as unreacted silane.

Preferably the surface is washed with a liquid comprising a solvent used in the modifying step. Agitation may also be employed during the washing step, if desired, e.g. using ultrasonic agitation methods.

It is also preferred that the modified surface is heated after the modifying step. Heating may also be carried out after the washing step, if a washing step is employed. This heat treatment has a number of advantageous effects, including a drying function, ensuring that volatile substances such as excess solvent are removed from the modified surface. In some embodiments of the present method, the heating step may also help to ensure that the modification reaction runs to completion. The degree of heating is not especially limited, provided that the heating is not deleterious to the advantageous properties of the modified surface outlined above. Typically the modified surface is heated to - a temperature of 50-200°C. More preferably the modified surface is heated to from 100-150°C.

In some aspects of the present method, the modification step itself may be carried out at an elevated temperature. This may be useful in decreasing the reaction time required for the modification step and ensuring that the modification reaction is as efficient as possible, requiring the minimum quantity of silane for achieving suitable levels of modification. The degree of heating employed is not strictly limited, and is dependent on the method of modification, the type of surface, and the particular silane compound employed. The heating-may include heating the surface, or heating the

silane, or heating both. When a solvent is employed, the solution of silane and solvent may be heated. In a preferred embodiment, when the method of modification employed involves immersion, the modification step is carried out by heating the silane solution to the desired temperature,- and immersing the unheated surface in the solution. Typically, the modification step is carried out at a temperature of 10-200°C. More preferably, the modification step is carried out at from ambient temperature to 100°C, such as from 15-100°C, from 20-100°C or from 25-100°C. When the surface to be modified is a plastic surface, it is preferred that the modification step is carried out at no more than 80°C.

When the surface to be modified is formed from a plastics material or a polymeric material, such as PBT or acetal, it is preferred in some embodiments of the invention to coat the plastics surface with aluminium before in turn modifying the aluminium surface. Aluminium can be deposited onto plastics materials using a sputter coating method, (e.g. with an Edwards S105B DC sputter coater). An aluminium layer a few nanometres thick can be deposited in this way. Such thicknesses are sufficient for the purposes of the present invention.

The fluorine-containin silane compound of formula (I) or (II) is not especially limited, provided that it is capable of modifying the surface to reduce the affinity of the surface to substances, such as pharmaceuticals, which may come into contact with it. Generally, the fluorine-containing silane compound comprises at least one fluorine-containing group which has a fluorinated region at some position within the group. Preferably, the group has a fluorinated terminus. By fluorinated, it is meant partial or complete substitution of a region of the group with fluorine atoms. By terminus, it is meant that portion of the group which contains the carbon atom that is furthest from the silicon atom of the silane to which the group is attached (furthest may mean furthest in absolute distance, or furthest when measured in number or length of bonds). The terminus need not consist only of the furthest carbon atom, and should merely comprise this atom. Thus the terminus may also be a group or region of carbon atoms, such as the last two, three, four, five or more carbon atoms in the group. If the group is branched it may have two or more terminal carbon atoms, each either equally distant or at differing distances from the silicon atom of the silane. In this case one or more of the terminal carbon atoms may be fluorinated. Preferably, the most distant terminal is fluorinated.

Groups comprising a fluorinated region are preferred, since the fluorinated region can form an inert barrier on the surface after modification has taken place. Generally the groups having a fluorinated region comprise hydrocarbon groups (preferably alkyl groups). When the fluorinated region is at the terminus of the group, it is preferred that the group is one in which at least the terminal carbon atom is fully substituted by fluorine atoms, i.e. the terminus comprises a $-CF_3$ group. Irrespective of whether the fluorinated region is at the terminus of the group or elsewhere, the other carbon atoms in the group may also be substituted with fluorine atoms, if desired, either partially or fully.

Preferred fluorinated termini comprised in the fluorine-containing group include those of the type $-(CF_2)_nCF_3$. Typically n is an integer from 1-20, more preferably 3-10. Particularly preferred termini include $-CF_3$, $-CF_2CF_3$, $CF_2CF_2CF_3$, $-CF_2CF_2CF_2CF_3$ and $CF_2CF_2CF_2CF_2CF_3$.

Provided that the silane compound is capable of achieving the desired effects, the degree or pattern of fluorination is not limited. Thus, as already mentioned above, in some embodiments, the fluorine atom or atoms need not be situated at the terminus of the fluorine-containing group. The fluorinated region may, for example be at any point between the terminus of the group and the silicon atom to which the group is attached. Thus the fluorine-containing group may also comprise non-fluorinated termini attached to the silicon atom via moieties comprising, for example, the following groups: $-CF_2-$, $-CF_2CF_2-$, $-CF_2CF_2CF_2-$ or $-CF_2CF_2CF_2CF_2-$.

Moreover, the fluorinated region need not be fully substituted with fluorine atoms, and may be interspersed with hydrogen atom substituents, or other substituents, if desired. Thus, the fluorine-containing group may also comprise termini such as the following: $-CHF_2$, $-CH_2F$, $-CHFCF_3$, $-CHFCHF_2$, $-CHFCH_2F$, $-CF_2CHF_2$ and $-CF_2CH_2F$; and/or may be attached to the silicon atom via moieties comprising groups such as the following: $-CHF-$, $-CHFCF_2-$, $-CHFCHF-$, or $-CF_2CHF-$.

In addition, further substituents other than fluorine atoms may be present in the fluorine-containing group, either within the fluorinated region, or elsewhere in the group. These further substituents are not particularly limited and may comprise any organic group.

When the further substituent comprises an organic group, the organic group preferably comprises a hydrocarbon group. The hydrocarbon group may comprise a straight chain, a branched chain or a cyclic group. Independently, the hydrocarbon group may comprise an aliphatic or an aromatic group. Also independently, the hydrocarbon group may comprise a saturated or unsaturated group.

When the hydrocarbon comprises an unsaturated group, it may comprise one or more alkene functionalities and/or one or more alkyne functionalities. When the hydrocarbon comprises a straight or branched chain group, it may comprise one or more primary, secondary and/or tertiary alkyl groups. When the hydrocarbon comprises a cyclic group it may comprise an aromatic ring, an aliphatic ring, and/or fused ring derivatives of these groups. The cyclic group may thus comprise a benzene, naphthalene, anthracene, indene, and/or a fluorene group, as well as regioisomers of the above groups.

In respect of such further substituents, the number of carbon atoms in the hydrocarbon group is not especially limited, but preferably the hydrocarbon group comprises from 1-40 C atoms. The hydrocarbon group may thus be a

lower hydrocarbon (1-6 C atoms) or a higher hydrocarbon (7 C atoms or more, e.g. 7-40 C atoms). The number of atoms in the ring of the cyclic group is not especially limited, but preferably the ring of the cyclic group comprises from 3-10 atoms, such as 3, 4, 5, 6 or 7 atoms.

In addition, any further substituent may comprise a combination of two or more of the substituents and/or functional groups defined above.

Without being bound by theory, it is believed that the barrier of fluorine-containing groups reduces the free energy of the surface, in turn reducing the affinity of the surface for other species. In other words, the barrier reduces the availability of 'free bonds' on the surface, as well as reducing less direct attractive forces, such as van der Waals forces (dispersion forces) which could otherwise provide a mechanism for attaching or attracting molecules to the surface. The free energy of the surface thus provides a useful measure of the 'attractiveness' of the surface to other species, and may be employed in the present invention as a guide to the effectiveness of modification. The contact angle of water and organic hydrophobic droplets on a surface is related to the free energy of the surface, and is useful as an indirect measure of the free energy of a surface. This provides an effective test for the suitability of a particular modification. This will be discussed in more detail below.

In one embodiment of the present invention, the fluorine-containing silane compound is a compound of the following formula (I):

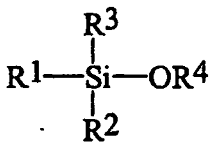

(I)

wherein $R^1$ comprises a substituted or unsubstituted straight chain or branched hydrocarbon group containing one or more fluorine atoms; and $R^2$, $R^3$ and $R^4$ are independently substituted or unsubstituted straight chain or branched hydrocarbon groups.

In the above formula, $R^1$ may be the fluorine-containing group already discussed in detail above, and may comprise a fluorine-containing region or regions as defined above. Preferably $R^1$ comprises a hydrocarbon group in which the terminus (e.g. first, second and/or third most distant carbon atom from the silicon atom) is substituted entirely by fluorine atoms. It is especially preferred that $R^1$ comprises an alkyl group. When $R^1$ comprises an alkyl group, the alkyl group may be a perfluoroalkyl group, i.e. a group in which all the hydrogen atoms are substituted with fluorine atoms. The alkyl group may be straight chain, or branched and thus may be a primary, secondary or tertiary alkyl group. The size of the $R^1$ group is not especially limited, provided that the silane compound is capable of successfully modifying the surface. Preferably $R^1$ comprises from 1-40 carbon atoms, and more preferably from 1-20 carbon atoms. More preferably still $R^1$ comprises from 3-15 carbon atoms, most preferably from 3-10 carbon atoms.

In the present invention $R^2$ and $R^3$ are not especially limited, provided that the silane compound is capable of successfully modifying the surface. Thus, in one embodiment of the invention $R^2$ and $R^3$ may independently be selected from the fluorine-containing groups as defined above in respect of $R^1$. In other embodiments of the present invention, only one of $R^2$ and $R^3$ comprises a fluorine containing group. Alternatively, and preferably, $R^2$ and $R^3$ independently comprise alkoxy groups or alkanoyloxy groups. When modifying an acetal surface, it is preferred that $R^2$ and $R^3$ both comprise alkoxy groups. The size of the $R^2$ and $R^3$ groups is not specifically limited. However, typically these groups independently comprise from 1-40 carbon atoms, preferably from 1-15 carbon atoms and more preferably from 1-10 carbon atoms. Preferably, when $R^2$ and/or $R^3$ comprise fluorine-containing groups, they are the same as $R^1$, and when they comprise alkoxy groups, they are the same as $OR^4$.

The $R^4$ group of the above formula is attached to the silicon atom via an oxygen atom. Without being bound by theory, it is believed (particularly for metallic surfaces) that the oxygen attached to this group serves as a linker from the silane compound to the surface. Generally, therefore, $R^4$ is selected such that the $OR^4$ group is capable of reacting with the surface to form such a linkage. Preferably $R^4$ comprises an alkyl group. The size of the $R^4$ group is not especially important in the present invention, although preferably $R^4$ comprises from 1-10 carbon atoms. It is particularly preferred that $R^4$ comprises methyl (Me) or ethyl (Et). In some embodiments, it is desirable to promote further linkages from the Si atom to the surface, in which case one or both of the $R^2$ and $R^3$ groups may comprise an oxygen atom attaching them to the silicon atom. In these embodiments the R2, $R^3$ and $OR^4$ may be identical, if desired.

In particularly preferred embodiments of the present invention, the fluorine-containing silane compound is selected from IH,IH,2H,2H-perfluorooctyltrimethoxysilane (MFS) IH, IH,2H,2H-perfluorooctyltriethoxysilane (EFS), 1H, 1H-perfluoroheptyltrimethoxysilane, 1,H,1H-perfluoroheptyltriethoxysilane, perfluorohexyltrimethoxysilane, perfluorohexyltriethoxysilane, perfluoropentyltrimethoxysilane perfluoropentyltriethoxysilane, perfluorobutyltrimethoxysilane, and

perfluorobutyltriethoxysilane.

In the present invention, the container preferably is a container suitable for storing a medicament. Thus, the container may be formed from any type of substance or material which is suitable for this purpose. The container may, for example, be formed from a metal, a plastic (polymeric) material, a glass or a ceramic, or other inert substance. Preferably the container is formed from a metal or a plastics material, such as a polymer. Typically, when the container is formed from a metal, the metal comprises aluminium or stainless steel. Typically, when the container is formed from a plastics material or a polymer it is formed from a polyformaldehyde resin (acetal), a polybutylene terephthalate (PBT), or a polyethylene terephthalate (PET). Preferably the container comprises a metal canister.

The above-discussed aspects of the present invention are particularly favoured for use with metal surfaces. The aspect of the present invention concerning modification of polymeric surfaces or plastics surfaces will now be discussed in more detail. Without being bound by theory it is believed that when the surface to be modified is comprised of a plastics or polymeric material, hydrogen bonding is important for attaching the fluorine-containing molecule to the surface. It is also likely that some chain entanglement is responsible for attaching molecules to a polymeric surface. Thus, when the surface to be modified is plastics or polymeric, it is desirable to employ a modifying agent which comprises a group capable of hydrogen bonding to the surface. Accordingly, the present invention also provides a method for modifying an internal polymeric surface of a container or closure, which comprises contacting the internal surface with a fluorine-containing silane compound of formula (II), which silane compound comprises a group capable of hydrogen bonding to the surface.

The group capable of hydrogen bonding is not especially limited, provided that the hydrogen bonding is strong enough to hold the modifying group on the surface. In a preferred embodiment, the group capable of hydrogen bonding to the surface (group Z in the Formulae below) comprises a hydroxy, carboxy and/or amino group.

In this aspect of the present invention, the fluorine-containing silane compound is a compound of Formula (II):

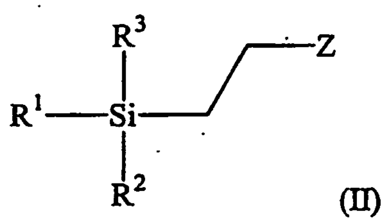

(II)

In addition to the silane compounds, a fluorine-containing carbon compound of Formula (III) can be used:

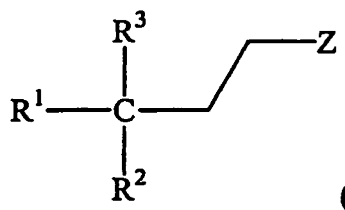

(III)

In formulas (II) and (III) Z comprises a group capable of hydrogen bonding to the surface, $R^1$ comprises a substituted or unsubstituted straight chain or branched hydrocarbon group containing one or more fluorine atoms; and $R^2$ and $R^3$ are independently substituted or unsubstituted straight chain or branched hydrocarbon groups. Preferably $R^1$ is a group as defined above in respect of the other aspects of the present invention. In the present invention $R^2$ and $R^3$ are not especially limited, provided that the compound is capable of successfully modifying the surface. Thus, in one embodiment of the invention $R^2$ and $R^3$ may independently be selected from the fluorine-containing groups as defined above in respect of $R^1$. In other embodiments of the present invention, none, or only one of $R^2$ and $R^3$ comprises a fluorine containing group. The size of the $R^2$ and $R^3$ groups is not specifically limited. However, typically these groups independently comprise from 1-40 carbon atoms, preferably from 1-15 carbon atoms and more preferably from 1-10 carbon atoms. Preferably, when $R^2$ and/or $R^3$ comprise fluorine-containing groups, they are the same as $R^1$, and when they comprise groups capable of hydrogen bonding, they are the same as the $-CH_2CH_2-Z$ group.

The most preferred fluorine-containing carbon compounds employed in this aspect of the present invention are $C_{10}F_{21}C(CH_2CH_2OH)_3$ and $C_8F_{17}C(CH_2CH_2NH_2)_3$.

In this aspect of the invention, any coating method can be employed, including the plasma coating and direct

coating (e.g. immersion) methods already discussed above.

In an additional aspect of the present invention, it is possible to further modify a surface (generally a plastics or polymeric surface) of a container or closure with a fluorine-containing carbon compound which may or may not comprise a group capable of hydrogen bonding. In this aspect of the invention, the process must involve a plasma coating method, as discussed above with reference to coating with fluorine-containing silane, compounds.

As mentioned above, in this aspect of the invention, the surface is usually a plastics surface (generally a polymeric surface) and the coating method is then preferably a cold plasma coating method as described above. In this aspect of the invention, the fluorine-containing carbon compound is preferably a gas or a liquid, such as a fluorine-containing solvent. Fluorine-containing compounds such as this may be partially fluorinated or fully fluorinated. These compounds preferably comprise from 1-10 carbon atoms, and are typically $C_1$-$C_{10}$ hydrocarbon compounds in which one or more of the hydrogen atoms have been substituted with fluorine atoms. More preferably, the compounds comprise from 1-6 carbon atoms and most preferably 1 or 2 carbon atoms. This is because these smaller compounds more easily form plasmas. Preferred compounds include $CF_4$, $CHF_3$, $CH_2F_2$, $CH_3F$, $CH_3CF_3$, $CH_3CHF_2$, and $CH_3CH_2F$.

In this aspect of the invention, the radio frequency used to generate the plasma is selected based upon the fluorine-containing carbon compound employed. For the preferred compounds mentioned above, such frequencies may be in the range 1 kHz to 1000 kHz, and are preferably from 10-100 kHz.

In the present method, an internal surface of the container or closure is modified. Naturally, this is because the pharmaceutical will contact at least one internal surface of the container. It is preferred, however, that the internal surface subjected to the modification step comprises substantially the entire internal surface of the container. This ensures that even if the pharmaceutical in the container contacts a surface that would not normally be contacted (e.g. through incorrect storage) there is no build-up of the pharmaceutical on this surface, nor is there any contamination of the pharmaceutical.

Preferably all internal surfaces which the pharmaceutical may come into contact with are modified. Generally the container, closure or canister is one comprising a cap and/or a metering unit comprising a valve, for dispensing a pharmaceutical. Thus, if the container is part of such a larger apparatus (a drug delivery system) then typically all internal surfaces in the system are modified, including passages designed to allow flow of the pharmaceutical, as well as valves, inlets, outlets and nozzles. Typically, containers modified according to the present method are employed in drug delivery systems such as dry powder inhalers (DPI), metered dose inhalers (MDI) and especially pressurised metered dose inhalers (pMDI). It is preferred that all of the internal surfaces of the DPI, MDI and pMDI systems which may come into contact with the pharmaceutical are modified. MDI systems generally comprise valves for regulating flow. These valves comprise valve components and rubbers, which are included in the valves to form seals. The valve components are generally formed from similar materials to the surfaces to be modified. Typically, the valve components are formed from metal (e.g. stainless steel and/or aluminium); acetal polymers (such as polyoxymethylene (POM)); acetal/PTFE (polytetrafluoroethylene) blends; acetal/PTFE/silicone blends; acetal/anti-static additive blends; PBT (poly-butylene terephthalate; PBT/PTFE blends; PBT/PTFE/silicone blends; PBT/anti-static additive blends; polyamides (such as nylon); and polyethylenes. Rubbers are typically formed from nitrile rubbers (e.g. butadiene-acrylonitrile); nitrile/PTFE blends; chloroprene (Neoprene®) rubber (polychloroprene); Neoprene®/PTFE blends; EPDM rubbers (ethylene-propylene-diene monomer terpolymers) or thermoplastic elastomers. In the present invention, the valve component and valve rubber surfaces may be modified in accordance with the present method if desired.

The present invention further provides a container for storing a medicament, which container comprises a modified internal surface, which surface comprises fluorine-containing hydrocarbon groups attached to the surface via a silicon-containing group, or via a group capable of hydrogen bonding to the surface wherein the surface has been treated with a silane of formula (I) or (II). The fluorine-containing hydrocarbon groups are as defined defined above in respect of the methods of the present invention, and in particular the groups $R^1$. The group capable of hydrogen bonding to the surface is a group Z as defined above in respect of the methods of the present invention.

The container may be formed from any suitable material, including the materials listed above for the containers employed in the present methods. Typically the container is comprised of a metal and/or a polymer. Preferably, the container is comprised of aluminium, stainless steel, a polyformaldehyde resin and/or a polybutylene terephthalate. The containers are typically metal canisters produced using a deep drawing or impact extrusion operation. If the canisters are to be anodised, aluminium alloy 5052 is preferably used to facilitate this. Stainless steel canisters are also available and may be modified with the agent addressed herein. Following deep drawing or impact extrusion, canisters are cleaned with an aliphatic hydrocarbon degreaser and surfactant, followed with a series of rinses with deionised water. In some processes, the canisters are then lightly anodised to produce a specific surface condition and high degree of cleanliness, without a trace of extractable organic compounds.

Anodising is preferably conducted using an electrochemical sulphuric acid Forest Products Laboratory (FPL) process with a carbon electrode. The canisters are connected to an alternating current source through a titanium clamp secured to the exterior surface of the neck. Anodising may take place with an applied current of 10 V AC, for a period of 5 minutes following immersion in sulphuric acid to produce an oxide layer with a specific microstructure approximately 0.8 $\mu$m in thickness. The preferred range is 0.6-0.9 $\mu$m. The canisters are next heat-sealed through immersion into a

water bath at 90°C, then rinsed in controlled purity water and dried with forced heated air convection. The thickness of the anodised layer may be measured by ultraviolet/visible light spectroscopic absorbence, calibrated against metallographic examination of representative anodised canister cross-sections.

Further provided by the present invention is a system for delivery of a medicament, comprising a container with a modified internal surface as defined above. In a preferred embodiment of the present invention the system comprises a metered dose inhaler (MDI or pMDI) for delivery of a medicament, such as a pulmonary or nasal medicament. Thus, an advantageous metered dose inhaler has been developed in the present invention for delivery of e.g. an active pulmonary or nasal medication. The system generally comprises a modified container obtainable using the process of the present invention described above. The inhaler is thus preferably comprised of a deep drawn aluminium alloy cylinder, modified with a silane added to limit drug adhesion, potentially adverse interaction with the aluminium canister and residual materials used for its production.

**Characterisation techniques**

In the present invention, a number of techniques for characterising the modified surfaces and the containers produced have been employed. These techniques are discussed in more detail below.

*X-ray photoelectron spectroscopy (XPS)*

In XPS a sample is irradiated with non-monochromatic X-rays, normally from aluminium or magnesium characteristic X-rays (e.g. Al $K_\alpha$ X-rays). The X-rays excite photoemission from the core levels of the atoms present in the sample and the resultant photoelectrons emerging from the sample surface are collected and energy analysed to yield the photoelectron spectrum. Since the kinetic energy of the photoelectron depends on the binding energy of electrons in the core levels from which photoemission is excited, each element gives rise to a set of peaks at characteristic energies in the photoelectron spectrum. Measurement of these energies therefore allows the elements present in the sample surface to be determined. Quantitative analysis is then obtained from the measurement of the relative intensities of the photoelectron peaks.

The inelastic mean free path of photoelectrons generated in a solid is typically 2-5 nm. This means that only those photoelectrons generated in the outermost atomic layers of the sample are likely to escape the surface, and be detected, with their initial kinetic energy intact. XPS is thus an analysis technique specific to the surface of solid materials, allowing routine analysis of the species present in amounts as small as 0.1 of a monolayer.

In addition to providing quantitative analysis of the outermost atomic layers of the sample, XPS may also be used to prove the chemistry of the surface. The binding energy of a core level depends principally on the charge on the nucleus of the atom concerned and to a lesser extent on the bonding between the atom and its neighbours. The change in binding energy due to chemical bonding is often referred to as the 'chemical shift'. Comparison between measured chemical shifts and published values for a wide variety of chemical compounds may allow the chemistry of the surface to be inferred and the functional groups present to be identified.

*Auger Electron Spectroscopy (AES)*

Auger electrons are produced by irradiating the surface of a material with a beam of electrons. The primary beams can ionise a surface atom leaving a hole in a core shell which is then filled by an electron from an outer shell; the excess energy of the atom can now be lost by two possible processes; the emission of a photon or the emission of an electron from an outer shell, the Auger electron. The energy of the Auger electron is determined by the difference in energy of the levels taking part in the process and is characteristic of the parent atom.

Auger electrons typically have energies in the range 0 to 2 keV. The escape depths of the electrons of this energy are only of the order of nanometres and consequently only electrons from the atoms in the top one or two atomic layers can escape from the surface without losing energy; Auger electrons originating from atoms in the bulk material interact with the surface atoms, thus losing energy, before escaping to add to the background. It is this property which gives the technique its surface sensitivity.

The instrument used in obtaining AES data for the present invention was the Varian Scanning Auger electron spectrometer. Its main features are as follows:

1. 10 keV, cylindrical mirror analyser system with 5 micron resolution.
2. Multiplex ion beam-depth profiling system (8 channel).
3. Scanner sample positioner with video display.
4. Auger chemical mapping system. This involves scanning the primary beam over the surface areas of interest and displaying the Auger signal of the element of interest on an oscilloscope screen as a function of beam position on

the sample.
5. Multiple sample handling facilities.

*Contact Angles And Surface Energies*

When the contact angle of a liquid on a solid (θ) is to be used to measure the surface energy of a solid, there are certain criteria that the solid must meet in order for the thermodynamic equations used in the calculation to be valid. For example, the sample must be atomically flat and chemically homogeneous. In reality, few samples meet this criteria and as a result there is not one unique contact angle for a particular solid. It is possible to measure an advancing angle and a receding angle. This difference between the two measured angles is called the hysteresis and is dependent on the deviations from ideality.
When dealing with real surfaces this hysteresis can yield some interesting information. For example, on relatively smooth but chemically heterogeneous samples with a significant hysteresis, e.g. >10°, the advancing angle is indicative of low surface energy regions whereas the receding angle is indicative of higher surface energy regions.
With samples exhibiting hysteresis and when θ is used for a surface energy calculation, it is important to state whether θ was an advancing or receding angle and the resulting surface energy be regarded as an estimation. When the hysteresis is much greater than 10° the estimation is not reliable at all.
In the present invention, contact angles were measured using a 'Krüss contact angle system' G40 (version 1.0 1987). A polar liquid (water) and an organic liquid (diiodomethane (DIM)) were employed. Water was triply distilled and diiodomethane was high purity grade. These liquids have surface tensions (y) as shown in the following Table 1:

Table 1

| Liquid | Surface tension at 20°C | | |
|---|---|---|---|
| | $\gamma_L$mNm$^{-1}$ | $\gamma_L{}^d$mNm$^{-1}$ | $\gamma_L{}^P$mNm$^{-1}$ |
| Water | 72.8 | 21.8 | 51.0 |
| DIM | 50.8 | 49.5 | 1.3 |

A drop of liquid is placed on the horizontal sample. An advancing contact angle is measured by increasing the volume of liquid via a syringe until the periphery moves along the sample surface. Immediately that the drop comes to rest the contact angle is measured.
A receding angle is-taken when volume is extracted from the drop causing the periphery to retreat back along the surface. Immediately at rest, the angle is measured.
An average is taken from 4 separate drops. Typical errors are ±2°.
On a chemically heterogeneous surface the advancing contact angle is representative of the least wettable regions within that surface, whereas the receding contact angle is representative of the most wettable regions.
From known reference values of the polar and dispersion components of surface energy of water and diiodomethane, and the contact angle θ of each liquid (1) on the unknown solid, a plot of x against y for the two liquids is made thus:

$$x = (\gamma_1{}^P/\gamma_1{}^d)^{1/2}$$

$$y = (1+\cos\theta)/2\cdot\gamma_1/(\gamma_1{}^d)^{1/2}$$

where
$\gamma_1{}^P$ = polar component of liquid's surface energy;
$\gamma_1{}^d$ = dispersion component of liquid's surface energy;
$\gamma_1$ = liquid surface energy.
From the linear plot the square of the gradient value is equivalent to $\gamma_s{}^P$, the polar component of the surface energy, and the square of the intercept value is equivalent to $\gamma_s{}^d$, the dispersion of component of surface energy. The total surface energy, $\gamma_s$, is given by $\gamma_s{}^d+\gamma_s{}^P$.
For the thermodynamic equations used in this method to be valid, surfaces must be atomically flat, chemically

homogenous and there must be no chemical interaction between the solid and liquid. In reality, the two former conditions are very rare and therefore, any surface energy calculated on common surfaces is only an estimation. (See D.K. Owens and RC. Wendt, J. Appl. Polym. Sci. 13, 1741, 1969, and R.J. Good, J. Adhesion Sci. Terminol., Vol. 6, No. 12 pp 1269-1302 (1992) for further contact angle and surface energy details which may be applied in the present invention).

The invention will now be described in further detail by way of example only, with reference to the following specific embodiments.

EXAMPLES

A study demonstrating the advantages of incorporating fluorine-containing silanes onto aluminium, acetal and polybutylene terephthalate (PBT) surfaces was performed.

The methodology for coating aluminium surfaces was then further optimised and applied to pMDI cans, which were filled with a hydrofluoroalkane (HFA) based suspension of a model drug. Adsorption of the drug onto the modified can walls was quantified and compared with adsorption onto unmodified cans.

Aluminium, acetal and PBT were identified as suitable test substrates, since they are commonly used for the construction of pMDI componentry.

*Example 1*

This Example intends to demonstrate the effect of modifying metal and plastics surfaces with fluorine-containing silanes. Sheets of aluminium and polybutylene terephthalate (PBT) were decontaminated by ultrasonic agitation in 1,1,2-trichloro-1,2,2-trifluoroethane (Freon® 113). Silane solutions were prepared by dissolving 0.5 g of fluoroalkyl silane in 1ml of Freon® 113. The silane moieties used for surface -modification were 1H,1H,2H,2H-perfluoroooctyltrimethoxysilane (MFS) and 1H,1H,2H,2H-perfluorooctyltriethoxysilane (EFS). The Freon® 113 solution was diluted with 94 ml methanol, followed by 5 ml 1M acetic acid. The substrate was immersed in the solution for 5 min, after which it was removed at a rate of 0.5 mm/min. The polymers and aluminium were then heated for 10 min at 120°C and 130°C respectively, washed with Freon® 113 for 5 mins with ultrasonic agitation to remove unreacted silane, and allowed to air dry. A fresh fluoroalkyl silane solution was prepared for each sheet of substrate.

X-ray photoelectron spectroscopy (XPS) analysis using Al $K_\alpha$ X-rays (VG ESCALAB spectrometry) and advancing and receding contact angle measurements using triply distilled water (G40 Goniometer) were used to characterise substrate surfaces. Results are shown in Figures 1 and 2.

It is apparent from Figures 1 and 2 that some fluoroalkyl silane groups were incorporated into the surfaces of the PBT substrates. This is indicated by a general increase in contact angles and in atom % of F by elemental analysis, when compared to untreated substrates. Treatment of aluminium appeared to be more effective, with a greater uptake of F and higher contact angles, indicating a lower surface energy. Because of the large hysteresis (the difference between advancing and receding contact angles) it was not meaningful to quantify surface energies. The observed hysteresis may have been caused by factors such as heterogeneity and roughness of the substrate surface.

*Example 2*

This Example is intended to demonstrate optimised conditions for the surface modification of aluminium. 30×10×0.47 mm aluminium strips were treated with the silane formulations detailed in Table 2, utilising the process described in Example 1.

Table 2

| **Components** | **Formulations** | | | |
|---|---|---|---|---|
| | **A** | **B** | **C** | **D** |
| MFS (g) | 0.5 | 0.25 | 0.25 | 0.25 |
| Freon® 113 (ml) | 1.0 | 0.5 | 0.5 | 0.5 |
| Methanol (ml) | 94.0 | 94.0 | | |
| 1M acetic acid (ml) | 5.0 | | | |
| Deionised water (ml) | | 6.0 | 90 | |
| 0.1M hydrochloric acid (ml) | | | 10 | |

(continued)

| Components | Formulations | | | |
|---|---|---|---|---|
| | A | B | C | D |
| Toluene (ml) | | | | 100 |

Surfaces were characterised by XPS and contact angle measurement with water. Results are shown in Figures 3 and 4.

Figures 3 and 4 indicate that a high drying temperature and the presence of 0.01 M hydrochloric acid to bring about acid hydrolysis of the fluoroalkyl silane and to activate the aluminium surface (Formulation C, as described in Table 2), produces the highest contact angle values and elemental F content.

*Example 3*

This Example aims to demonstrate the advantages of modifying the surface of an aluminium pMDI can. Rectangles (20×10 mm) were partially cut from the walls of 19 ml cans (Presspart, UK), leaving a bridge in place. These cans were decontaminated, together with additional intact cans, by ultrasonic agitation in Freon® 113. After drying, a sample can was taken as a control, and the remaining cans were submerged in Formulation C for 30 min. The cans were then removed, dried at room temperature, and then placed in an oven at 130°C for 10 min. They were then immersed in Freon® 113 for 5 min with ultrasonic agitation, and the partially cut rectangles removed for surface analysis by XPS (using an ESCALAB 5 device) and Auger Electron Spectroscopy (AES) (using a Varian Scanning Auger electron spectrometer).

Figure 5 indicates that pMDI cans were successfully treated with Formulation C, with both XPS and AES data indicating higher levels of F in treated cans compared to controls. AES results indicated lower F levels than XPS and data was more variable. This may be a result of decomposition of the fluoroalkyl silane, induced by the higher energy density of electrons used in this process, compared to the X-rays used in XPS analysis. Decomposition of organic materials during AES analysis has been reported previously (Briggs, 1985).

*Example 4*

This Example illustrates the effect of modification on drug storage. The silane treated pMDI cans of Example 3, together with untreated controls, were crimped with a metering valve (Valois, UK) and filled with an HFA based suspension formulation of a model drug (Drug A) using a pressure fill process. After a 28 day equilibration period the pMDI units were cooled to below the boiling point of the propellant, and the valves removed. The contents of the pMDI were emptied, and the cans washed with aliquots of propellant to remove unbound drug. The remaining, adsorbed drug was then dissolved in a suitable solvent and assayed by reverse phase HPLC.

The amount of Drug A deposited on the walls of treated aluminium pMDI cans (expressed as a percentage of total drug filled into cans), was less than that deposited onto untreated controls following a 28 day post filling equilibration period ($6.75 \pm 2.34$% and $10.73 \pm 3.64$ % respectively; means $\pm$ SD, n=4). This confirms that modification of the aluminium can surface is an excellent means of reducing drug deposition.

*Example 5*

This Example is intended to illustrate the different effects of utilising different coating methods on plastics surfaces. The effects of direct coating (immersion) and, direct coating after aluminium deposition were investigated. For comparison with the results of Example 5, it should be noted that the surface composition (in atom %, excluding H) and average advanced water contact angle (WCA) in degrees for the untreated acetal and PBT are as follows:

Table 3 - non treated materials

| Material | C | O | Si | F | WCA |
|---|---|---|---|---|---|
| Acetal | 59.3 | 40.7 | 0 | 0 | 78 |
| PBT | 80.4 | 19.6 | 0 | 0 | 84 |

*Comparison of direct coating directly onto acetal and PBT and direct coating onto the same plastics after aluminium deposition*

In this test, solutions of 0.5 g or 1.5 g of 1H,1H,2H,2H-perfluoroethyltrimethoxysilane in 94 ml and 93 ml methanol respectively were employed. Each solution also comprised 5 ml 1M acetic acid. The silane solutions were aged for 30 minutes at room temperature. Strips of acetal and PBT were then immersed in the solutions for 5 minutes. The plastics were then dried with a tissue, prior to heating to 80°C or 120°C in an oven for 30 minutes. On cooling, the strips were placed in a beaker containing 1,1,2-trichlorotrifluoroethane and cleaned by ultrasonic agitation for five minutes.

For aluminium coating, aluminium was deposited onto acetal and PBT strips using a DC sputter coating device (Edwards S150B). The aluminium coating achieved was a few nanometres in thickness. The presence of aluminium was confirmed with a zero water contact angle, which compared with values of 78° and 84° for the untreated acetal and PBT respectively. The coated plastics were then treated with the silane solutions as described above.

The C, O, Si and F content (in atom %, excluding H) of the resulting surfaces were measured using XPS. In addition, the average advanced water contact angle (WCA) in degrees was also measured. The results are shown in Tables 4 and 5 below.

Table 4 - direct coating

| Material | Solution strength/baking temp | C | O | Si | F | WCA |
|---|---|---|---|---|---|---|
| Acetal | 0.5 %/80°C | 61.6 | 35.9 | 0.3 | 2.1 | 84 |
| Acetal | 0.5 %/120°C | 63.1 | 35.4 | 0.4 | 1.0 | 103 |
| Acetal | 1.5 %/80°C | 58.8 | 33.9 | 1.0 | 6.4 | 95 |
| Acetal | 1.5 %/120°C | 59.4 | 34.1 | 0.4 | 6.2 | 86 |
| PBT | 0.5 %/80°C | 76.6 | 18.7 | 0.7 | 2.9 | 90 |
| PBT | 0.5 %/120°C | 77.0 | 17.4 | 0.9 | 4.6 | 90 |
| PBT | 1.5 %/80°C | 70.2 | 15.9 | 1.3 | 12.6 | 105 |
| PBT | 1.5 %/120°C | 69.5 | 15.7 | 1.8 | 12.9 | 108 |

Table 5 - direct coating after aluminium deposition

| Material | Solution strength/baking temp | C | O | Si | F | WCA |
|---|---|---|---|---|---|---|
| Acetal | 0.5 %/80°C | 45.4 | 27.4 | 2.9 | 14.0 | 142 |
| Acetal | 0.5 %/ 120°C | 48.6 | 26.8 | 2.1 | 11.8 | 141 |
| Acetal | 1.5 %/80°C | 34.6 | 29.0 | 2.0 | 25.8 | 138 |
| Acetal | 1.5 %/120°C | 45.6 | 28.4 | 2.3 | 13.6 | 140 |
| PBT | 0.5 %/80°C | 45.0 | 22.5 | 2.9 | 20.5 | 112 |
| PBT | 0.5 %/120°C | 42.8 | 24.3 | 3.5 | 18.9 | 128 |
| PBT . | 1.5 %/80°C | 32.6 | 27.0 | 4.3 | 26.7 | 132 |
| PBT | 1.5 %/120°C | 44.8 | 23.8 | 3.4 | 19.4 | 151 |

In the above tests it can clearly be seen that the surface is modified by the introduction of fluorine-containing silane, which leads to an increase in average advanced water contact angle (WCA). The degree and effectiveness varies according to the strength of solution used and temperature of baking. Additionally, coating the plastics with aluminium can also be seen to increase the effectiveness of modification.

In summary, the utility of modifying the surfaces of materials utilised in pMDI componentry with fluoroalkyl silanes, such that surface energies are reduced has been demonstrated. XPS and contact angle measurement have been successfully used to characterise the substrate surfaces in these studies. The treatment process has been optimised for aluminium pMDI cans and it has been demonstrated that this results in reduced deposition of a model drug.

The above results demonstrate that the process of the present invention produces containers having superior internal surfaces. This leads to the advantage that container contents, such as medicaments, adhere less to the containers. Surface modification therefore presents an alternative, improved approach to known methods of reducing drug adsorption onto pMDI componentry.

## Claims

1. A method for modifying an internal surface of a container or closure, which method comprises contacting the internal surface with a fluorine-containing silane compound to form a modified surface,
wherein
the fluorine-containing silane compound is a compound of the following formula (I):

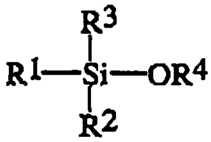

wherein $R^1$ comprises a substituted or unsubstituted straight chain or branched hydrocarbon group containing one or more fluorine atoms; and $R^2$, $R^3$ and $R^4$ are independently substituted or unsubstituted straight chain or branched hydrocarbon groups, or
the fluorine-containing silane compound is a compound of Formula (11):

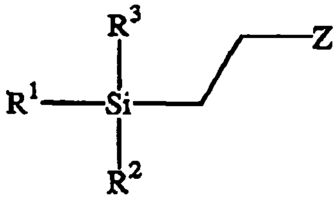

(II)

wherein Z comprises a group capable of hydrogen bonding to the surface, $R^1$ comprises a substituted or unsubstituted straight chain or branched hydrocarbon group containing one or more fluorine atoms; and $R^2$ and $R^3$ are independently substituted or unsubstituted straight chain or branched hydrocarbon groups.

2. A method according to claim 1, wherein prior to modifying, the surface to be modified is prepared for modification by cleaning the surface to be modified.

3. A method according to claim 2, wherein preparation comprises contacting the surface to be modified with hydrochloric acid

4. A method according to any preceding claim, wherein after modifying, the modified surface is heated.

5. A method according to claim 4, wherein the modified surface is heated to a temperature of 50-200°C.

6. A method according to any preceding claim, wherein modifying is carried out at a temperature of 15-100°C.

7. A method according to any preceding claim, wherein modifying comprises immersing the surface to be modified in a solution of the fluorine-containing silane compound.

8. A method according to claim 7, wherein the immersion is carried out for 10 mins or less.

9. A method according to any of claims 1-6, wherein modifying comprises applying a plasma coating step to the surface.

10. A method according to any preceding claim, wherein the modifying is repeated one or more times.

**11.** A method according to any preceding claim, wherein the fluorine-containing silane compound comprises a group attached to the silicon atom of the silane, which group is fluorinated at its terminus.

**12.** A method according to Claim 11, wherein the fluorinated terminus comprised in the fluorine-containing group has the formula $-(CF_2)_nCF_3$, wherein n is an integer from 1-20.

**13.** A method according to any preceding claim, wherein $R^1$ in formula (I) comprises a hydrocarbon group in which the first, second and/or third most distant carbon atom from the silicon atom is (are) substituted entirely by fluorine atoms.

**14.** A method according to any preceding claim, wherein $R^1$ in formula (I) comprises an alkyl group.

**15.** A method according to claim 14, wherein $R^1$ comprises a perfluoroalkyl group.

**16.** A method according to any any preceding claim, wherein $R^2$ and $R^3$ in formula (I) independently comprise alkoxy groups or alkanoyloxy groups.

**17.** A method according to any any preceding claim, wherein $R^2$ and $R^3$ in formula (I) independently comprise from X-10 carbon atoms.

**18.** A method according to any any preceding claim, wherein $R^4$ in formula (I) comprises an alkyl group.

**19.** A method according to claim 18, wherein $R^4$ comprises from 1-10 carbon atoms.

**20.** A method according to any any preceding claim, wherein the $R^2$, $R^3$ and $OR^4$ groups in formula (I) are identical.

**21.** A method according to any any preceding claim, wherein $R^1$ in formula (I) comprises from 1-20 carbon atoms.

**22.** A method according to claim 11, wherein the fluorine-containing silane compound is selected from 1H, 1H,2H,2H-perfluorooctyltrimethoxysilane (MFS) and 1H,1H,2H,2H-perfluorooctyltriethoxysilane (EFS).

**23.** A method according to any preceding claim, wherein the container comprises a metal canister, or is comprised of a plastics material or a polymer.

**24.** A method according to claim 23, wherein the container is comprised of a plastics material or a polymer and prior to modifying the surface to be modified is coated with aluminium.

**25.** A method according to claim 23, wherein the container is comprised of metal and the metal comprises aluminium or stainless steeL

**26.** A method according to any preceding claim, wherein the internal surface subjected to modifying comprises substantially the entire internal surface of the container or closure.

**27.** A method according to any preceding claim, wherein the silane compound comprises a group capable of hydrogen bonding to the surface.

**28.** A method according to claim 27, wherein the group capable of hydrogen bonding to the surface comprises a hydroxy, carboxy and/or amino group.

**29.** A method according to any preceding claim, wherein $R^2$ and/or $R^3$ in Formula (II) are identical to the $-CH_2CH_2-Z$ group.

**30.** A method according to any any preceding claim, wherein additionally a fluorine-containing carbon compound of formula $C_{10}F_{21}C(CH_2CH_2OH)_3$ or $C_8F_{17}C(CH_2CH_2NH_2)_3$ is used.

**31.** A method according to any preceding claim, comprising contacting the internal surface with a fluorine-containing carbon compound in a plasma coating step.

**32.** A method according to 31, wherein the fluorine-containing carbon compound is a $C_1$-$C_{10}$ hydrocarbon compound in which one or more of the hydrogen atoms have been substituted with fluorine atoms.

**33.** A method according to claim 32, wherein the fluorine-containing carbon compound comprises a compound selected from $CF_4$, $CHF_3$, $CH_2F_2$, $CH_3F$, $CH_3CF_3$, $CH_3CHF_2$. and $CH_3CH_2F$.

**34.** A container for storing a medicament, which container comprises a modified internal surface, which surface comprises fluorine-containing hydrocarbon groups bonded to the surface via a silicon-containing group,
wherein the surface has been treated with a
fluorine-containing silane compound of the following formula (I):

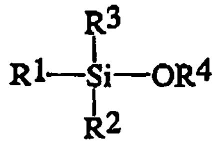

(I)

wherein $R^1$ comprises a substituted or unsubstituted straight chain or branched hydrocarbon group containing one or more fluorine atoms; and $R^2$, $R^3$ and $R^4$ are independently substituted or unsubstituted straight chain or branched hydrocarbon groups, or with a
fluorine-containing silane compound of Formula (II):

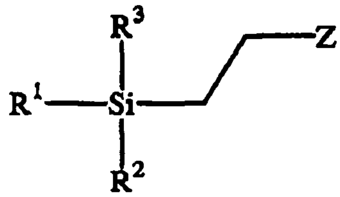

(II)

wherein Z comprises a group capable of hydrogen bonding to the surface, $R^1$ comprises a substituted or unsubstituted straight chain or branched hydrocarbon group containing one or more fluorine atoms; and $R^2$ and $R^3$ are independently substituted or unsubstituted straight chain or branched hydrocarbon groups.

**35.** A container according to claim 34, wherein the fluorine-containing hydrocarbon groups comprise groups $R^1$ as defined in any of claims 13-15 or 21.

**36.** A container according to claims 34 or 35, which container is comprised of a metal and/or a plastics or polymer material.

**37.** A container according to claim 36, which container is comprised of aluminium, stainless steel, a polyformaldehyde resin and/or a polybutylene terephthalate.

**38.** A system for delivery of a medicament, comprising a container with a modified internal surface as defined in any of claims 34-37.

**39.** A system according to claim 38, comprising a metered dose inhaler (MDI) or a pressurised metered dose inhaler (pMDI) for delivery of a pulmonary or nasal medicament.

## Patentansprüche

**1.** Verfahren zur Modifizierung einer Innenfläche eines Behälters oder Verschlusses, wobei bei dem Verfahren die Innenfläche mit einer fluorhaltigen Silanverbindung zur Bildung einer modifizierten Oberfläche in Berührung gebracht wird, wobei die fluorhaltige Silanverbindung eine Verbindung der folgenden Formel (I) ist:

$$R^1 - \underset{R^2}{\overset{R^3}{Si}} - OR^4$$

wobei $R^1$ eine substituierte oder unsubstituierte geradkettige oder verzweigte Kohlenwasserstoffgruppe mit einem oder mehr Fluoratomen umfasst; und $R^2$, $R^3$ und $R^4$ unabhängig voneinander substituierte oder unsubstituierte geradkettige oder verzweigte Kohlenwasserstoffgruppen sind, oder
die fluorhaltige Silanverbindung eine Verbindung der Formel (II) ist:

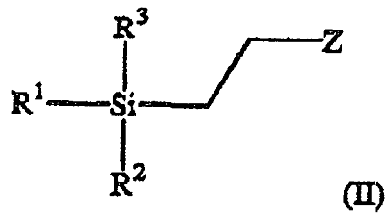

(II)

wobei Z eine Gruppe umfasst, die Wasserstoff an der Oberfläche binden kann, $R^1$ eine substituierte oder unsubstituierte geradkettige oder verzweigte Kohlenwasserstoffgruppe mit ein oder mehr Fluoratomen umfasst; und $R^2$ und $R^3$ unabhängig voneinander substituierte oder unsubstituierte geradkettige oder verzweigte Kohlenwasserstoffgruppen sind.

**2.** Verfahren nach Anspruch 1, wobei vor der Modifizierung die zu modifizierende Oberfläche durch Reinigung der zu modifizierenden Oberfläche auf die Modifizierung vorbereitet wird.

**3.** Verfahren nach Anspruch 2, wobei bei der Vorbereitung die zu modifizierende Oberfläche mit Salzsäure in Berührung gebracht wird.

**4.** Verfahren nach einem der vorhergehenden Ansprüche, wobei die modifizierte Oberfläche nach der Modifizierung erhitzt wird.

**5.** Verfahren nach Anspruch 4, wobei die modifizierte Oberfläche auf eine Temperatur von 50-200°C erhitzt wird.

**6.** Verfahren nach einem der vorhergehenden Ansprüche, wobei die Modifizierung bei einer Temperatur von 15-100°C durchgeführt wird.

**7.** Verfahren nach einem der vorhergehenden Ansprüche, wobei bei der Modifizierung die zu modifizierende Oberfläche in eine Lösung aus einer fluorhaltigen Silanverbindung eingetaucht wird.

**8.** Verfahren nach Anspruch 7, wobei der Eintauchvorgang 10 Minuten oder weniger dauert.

**9.** Verfahren nach einem der Ansprüche 1-6, wobei die Oberfläche bei der Modifizierung einem Plasmabeschichtungsschritt unterzogen wird.

**10.** Verfahren nach einem der vorhergehenden Ansprüche, wobei die Modifizierung ein- oder mehrmals wiederholt wird.

**11.** Verfahren nach einem der vorhergehenden Ansprüche, wobei die fluorhaltige Silanverbindung eine Gruppe umfasst, die an dem Silikonatom des Silans befestigt ist, wobei die Gruppe an ihrem Ende fluoriert ist.

**12.** Verfahren nach Anspruch 11, wobei das fluorierte Ende in der fluorhaltigen Gruppe die Formel $-(CF_2)_nCF_3$ hat, wobei n eine ganze Zahl von 1-20 ist.

**13.** Verfahren nach einem der vorhergehenden Ansprüche, wobei $R^1$ in Formel (I) eine Kohlenwasserstoffgruppe umfasst, in der das erste, zweite und/oder dritte am weitesten vom Silikonatom entfernte Kohlenstoffatom vollständig durch Fluoratome substituiert ist.

**14.** Verfahren nach einem der vorhergehenden Ansprüche, wobei $R^1$ in Formel (I) eine Alkylgruppe umfasst.

**15.** Verfahren nach Anspruch 14, wobei $R^1$ eine Perfluoralkylgruppe umfasst.

**16.** Verfahren nach einem der vorhergehenden Ansprüche, wobei $R^2$ und $R^3$ in Formel (I) unabhängig voneinander Alkoxygruppen oder Alkanoyloxy-Gruppen umfassen.

**17.** Verfahren nach einem der vorhergehenden Ansprüche, wobei $R^2$ und $R^3$ in Formel (I) unabhängig voneinander 1-10 Kohlenstoffatome umfassen.

**18.** Verfahren nach einem der vorhergehenden Ansprüche, wobei $R^4$ in Formel (I) eine Alkylgruppe umfasst.

**19.** Verfahren nach Anspruch 18, wobei $R^4$ 1-10 Kohlenstoffatome umfasst.

**20.** Verfahren nach einem der vorhergehenden Ansprüche, wobei die Gruppen $R^2$, $R^3$ und $OR^4$ in Formel (I) identisch sind.

**21.** Verfahren nach einem der vorhergehenden Ansprüche, wobei $R^1$ in Formel (I) 1-20 Kohlenstoffatome umfasst.

**22.** Verfahren nach Anspruch 11, wobei die fluorhaltige Silanverbindung unter 1H,1H,2H,2H-Perfluoroctyltrimethoxysilan (MFS) und 1H,1H,2H,2H-Perfluoroctyltriethoxysilan (EFS) ausgewählt ist.

**23.** Verfahren nach einem der vorhergehenden Ansprüche, wobei der Behälter einen Metallkanister umfasst oder aus einem Plastikmaterial oder aus einem Polymer besteht.

**24.** Verfahren nach Anspruch 23, wobei der Behälter aus einem Plastikmaterial oder einem Polymer besteht und die zu modifizierende Oberfläche vor der Modifizierung mit Aluminium beschichtet wird.

**25.** Verfahren nach Anspruch 23, wobei der Behälter aus Metall besteht und das Metall Aluminium oder Edelstahl umfasst.

**26.** Verfahren nach einem der vorhergehenden Ansprüche, wobei die der Modifizierung unterworfene Innenfläche im Wesentlichen die gesamte Innenfläche des Behälters oder Verschlusses umfasst.

**27.** Verfahren nach einem der vorhergehenden Ansprüche, wobei die Silanverbindung eine Gruppe umfasst, die Wasserstoff an der Oberfläche binden kann.

**28.** Verfahren nach Anspruch 27, wobei die Gruppe, die Wasserstoff an der Oberfläche binden kann, eine Hydroxy-, Carboxy- und/oder Aminogruppe umfasst.

**29.** Verfahren nach einem der vorhergehenden Ansprüche, wobei $R^2$ und/oder $R^3$ in Formel (II) mit der $-CH_2CH_2-Z-$Gruppe identisch sind.

**30.** Verfahren nach einem der vorhergehenden Ansprüche, wobei zusätzlich eine fluorhaltige Kohlenstoffverbindung der Formel $C_{10}F_{21}C\ (CH_2CH_2OH)_3$ oder $C_8F_{17}C(CH_2CH_2NH_2)_3$ verwendet wird.

**31.** Verfahren nach einem der vorhergehenden Ansprüche, wobei die Innenfläche mit einer fluorhaltigen Kohlenstoffverbindung in einem Plasmabeschichtungsschritt in Berührung gebracht wird.

**32.** Verfahren nach Anspruch 31, wobei die fluorhaltige Kohlenstoffverbindung eine $C_1$-$C_{10}$-Kohlenwasserstoffverbindung ist, in der eines oder mehr der Wasserstoffatome durch Fluoratome substituiert sind.

**33.** Verfahren nach Anspruch 32, wobei die fluorhaltige Kohlenstoffverbindung eine Verbindung umfasst, die unter $CF_4$, $CHF_3$, $CH_2F_2$, $CH_3F$, $CH_3CF_3$, $CH_3CHF_2$ und $CH_3CH_2F$ ausgewählt wurde.

**34.** Behälter zur Aufbewahrung eines Medikaments, der eine modifizierte Innenfläche umfasst, wobei diese Oberfläche fluorhaltige Kohlenwasserstoffgruppen umfasst, die über eine silikonhaltige Gruppe an die Oberfläche gebunden sind, wobei die Oberfläche mit einer fluorhaltigen Silanverbindung der folgenden Formel (I):

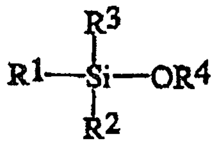

(I)

wobei $R^1$ eine substituierte oder unsubstituierte geradkettige oder verzweigte Kohlenwasserstoffgruppe mit einem oder mehr Fluoratomen umfasst; und $R^2$, $R^3$ und $R^4$ unabhängig voneinander substituierte oder unsubstituierte geradkettige oder verzweigte Kohlenwasserstoffgruppen sind, oder mit einer fluorhaltigen Silanverbindung der Formel (II) behandelt wurde:

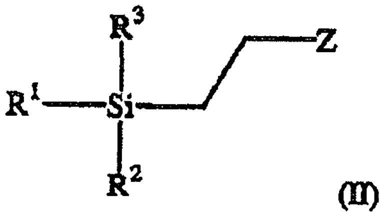

(II)

wobei Z eine Gruppe umfasst, die Wasserstoff an der Oberfläche binden kann, $R^1$ eine substituierte oder unsubstituierte geradkettige oder verzweigte Kohlenwasserstoffgruppe mit einem oder mehr Fluoratomen umfasst; und $R^2$ und $R^3$ unabhängig voneinander substituierte oder unsubstituierte geradkettige oder verzweigte Kohlenwasserstoffgruppen sind.

**35.** Behälter nach Anspruch 34, wobei die fluorhaltigen Kohlenwasserstoffgruppen $R^1$ wie in einem der Ansprüche 13-15 oder 21 definiert umfassen.

**36.** Behälter nach Anspruch 34 oder 35, wobei der Behälter aus einem Metall und/oder einem Plastik- oder Polymermaterial besteht.

**37.** Behälter nach Anspruch 36, wobei der Behälter aus Aluminium, Edelstahl, einem Polyformaldehydharz und/oder einem Polybutylenterephthalat besteht.

**38.** System zur Abgabe eines Medikaments, umfassend einen Behälter mit einer modifizierten Innenfläche wie in einem der Ansprüche 34-37 definiert.

**39.** System nach Anspruch 38, umfassend ein Dosieraerosol (MDI) oder ein unter Druck stehendes Dosieraerosol (pMDI) zur Abgabe eines pulmonalen oder nasalen Medikaments.

## Revendications

**1.** Procédé de modification d'une surface interne d'un récipient ou d'un couvercle, lequel procédé comprend la mise en contact de la surface interne avec un composé de silane comprenant du fluor pour former une surface modifiée, dans lequel
le composé de silane comprenant du fluor est un composé de la formule (I) suivante:

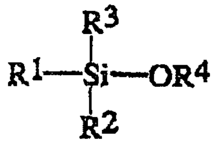

dans laquelle $R^1$ comprend un groupe d'hydrocarbures ramifiés ou à chaîne droite, substitué ou non substitué, qui comprend un ou plusieurs atomes de fluor ; et $R^2$, $R^3$ et $R^4$ sont indépendamment des groupes d'hydrocarbures ramifiés ou à chaîne droite, substitués ou non substitués, ou
le composé de silane comprenant du fluor est un composé de la formule (II) :

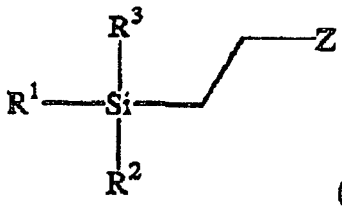

(II)

dans laquelle Z comprend un groupe capable d'établir une liaison hydrogène avec la surface, $R^1$ comprend un groupe d'hydrocarbures ramifiés ou à chaîne droite, substitué ou non substitué, qui comprend un ou plusieurs atomes de fluor ; et $R^2$ et $R^3$ sont indépendamment des groupes d'hydrocarbures ramifiés ou à chaîne droite, substitués ou non substitués.

2. Procédé selon la revendication 1, dans lequel la surface à modifier est préparée pour une modification en nettoyant la surface à modifier avant la modification.

3. Procédé selon la revendication 2, dans lequel la préparation comprend la mise en contact de la surface à modifier avec de l'acide chlorhydrique.

4. Procédé selon l'une quelconque des revendications précédentes, dans lequel la surface modifiée est chauffée après la modification.

5. Procédé selon la revendication 4, dans lequel la surface modifiée est chauffée à une température de 50 à 200 °C.

6. Procédé selon l'une quelconque des revendications précédentes, dans lequel la modification est effectuée à une température de 15 à 100 °C.

7. Procédé selon l'une quelconque des revendications précédentes, dans lequel la modification comprend l'immersion de la surface à modifier dans une solution du composé de silane comprenant du fluor.

8. Procédé selon la revendication 7, dans lequel l'immersion est effectuée pour une période de dix minutes ou moins.

9. Procédé selon l'une des revendications 1 à 6,
dans lequel la modification comprend une étape d'application à la surface d'un revêtement par projection plasma.

10. Procédé selon l'une quelconque des revendications précédentes, dans lequel la modification est répétée une ou plusieurs fois.

11. Procédé selon l'une quelconque des revendications précédentes, dans lequel le composé de silane comprenant du fluor comprend un groupe attaché à l'atome de silicium du silane, lequel groupe est fluoré à son extrémité.

12. Procédé selon la revendication 11, dans lequel l'extrémité fluorée comprise dans le groupe comprenant du fluor possède la formule $-(CF_2)_nCF_3$, dans laquelle n est un nombre entier de 1 à 20.

**13.** Procédé selon l'une quelconque des revendications précédentes, dans lequel $R^1$ dans la formule (I) comprend un groupe d'hydrocarbures dans lequel le premier, le deuxième et/ou le troisième atome de carbone le plus distant de l'atome de silicium est (sont) substitué(s) complètement par des atomes de fluor.

**14.** Procédé selon l'une quelconque des revendications précédentes, dans lequel $R^1$ dans la formule (I) comprend un groupe alkyle.

**15.** Procédé selon la revendication 14, dans lequel $R^1$ comprend un groupe perfluoroalkyle.

**16.** Procédé selon l'une quelconque des revendications précédentes, dans lequel $R^2$ et $R^3$ dans la formule (I) comprennent indépendamment des groupes alcoxy ou des groupes alcanoyloxy.

**17.** Procédé selon l'une quelconque des revendications précédentes, dans lequel $R^2$ et $R^3$ dans la formule (I) comprennent indépendamment de 1 à 10 atomes de carbone.

**18.** Procédé selon l'une quelconque des revendications précédentes, dans lequel $R^4$ dans la formule (I) comprend un groupe alkyle.

**19.** Procédé selon la revendication 18, dans lequel $R^4$ comprend de 1 à 10 atomes de carbone.

**20.** Procédé selon l'une quelconque des revendications précédentes, dans lequel $R^2$ $R^3$ et le groupe $OR^4$ dans la formule (I) sont identiques.

**21.** Procédé selon l'une quelconque des revendications précédentes, dans lequel $R^1$ dans la formule (I) comprend de 1 à 20 atomes de carbone.

**22.** Procédé selon la revendication 11, dans lequel le composé de silane comprenant du fluor est choisi parmi le 1H, 1H,2H,2H-perfluorooctyltriméthoxysilane (MFS) et le 1H,1H,2H,2H-perfluorooctyltriéthoxysilane (EFS).

**23.** Procédé selon l'une quelconque des revendications précédentes, dans lequel le récipient comprend une boîte métallique, ou est composé d'un matériau en plastique ou d'un polymère.

**24.** Procédé selon la revendication 23, dans lequel le récipient est composé d'un matériau en plastique ou d'un polymère et, avant la modification, la surface à modifier est recouverte d'aluminium.

**25.** Procédé selon la revendication 23, dans lequel le récipient est composé de métal et le métal comprend de l'aluminium ou de l'acier inoxydable.

**26.** Procédé selon l'une quelconque des revendications précédentes, dans lequel la surface interne soumise à la modification comprend essentiellement toute la surface interne du récipient ou du couvercle.

**27.** Procédé selon l'une quelconque des revendications précédentes, dans lequel le composé de silane comprend un groupe capable de former une liaison hydrogène avec la surface.

**28.** Procédé selon la revendication 27, dans lequel le groupe capable de former une liaison hydrogène avec la surface comprend un groupe hydroxy, un groupe carboxy et/ou un groupe amino.

**29.** Procédé selon l'une quelconque des revendications précédentes, dans lequel $R^2$ et/ou $R^3$ dans la formule (II) sont identiques au groupe $-CH_2CH_2-Z$.

**30.** Procédé selon l'une quelconque des revendications précédentes, dans lequel on utilise en outre un composé de carbone comprenant du fluor de la formule $C_{10}F_{21}C\ (CH_2CH_2OH)_3$ ou $C_8F_{17}C(CH_2CH_2NH_2)_3$.

**31.** Procédé selon l'une quelconque des revendications précédentes, qui comprend la mise en contact de la surface interne avec un composé de carbone comprenant du fluor dans une étape de revêtement par projection plasma.

**32.** Procédé selon la revendication 31, dans lequel le composé de carbone comprenant du fluor est un composé d'hydrocarbures de 1 à 10 atomes de carbone dans lequel un ou plusieurs des atomes d'hydrogène ont été substitués

par des atomes de fluor.

**33.** Procédé selon la revendication 32, dans lequel le composé de carbone comprenant du fluor comprend un composé choisi parmi le $CF_4$, le $CHF_3$, le $CH_2F_2$, le $CH_3F$, le $CH_3CF_3$, le $CH_3CHF_2$ et le $CH_3CH_2F$.

**34.** Récipient pour l'entreposage d'un médicament, lequel récipient comprend une surface interne modifiée, laquelle surface comprend des groupes d'hydrocarbures comprenant du fluor liés à la surface par l'intermédiaire d'un groupe comprenant du silicium,
dans lequel la surface a été traitée avec un composé de silane comprenant du fluor de la formule (I) suivante :

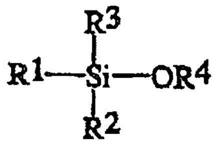

(I)

dans laquelle $R^1$ comprend un groupe d'hydrocarbures ramifiés ou à chaîne droite, substitué ou non substitué, qui comprend un ou plusieurs atomes de fluor ; et $R^2$, $R^3$ et $R^4$ sont indépendamment des groupes d'hydrocarbures ramifiés ou à chaîne droite, substitués ou non substitués, ou avec un composé de silane comprenant du fluor de la formule (II) :

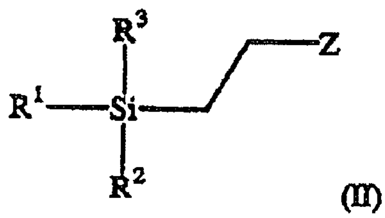

(II)

dans laquelle Z comprend un groupe capable d'établir une liaison hydrogène avec la surface, $R^1$ comprend un groupe d'hydrocarbures ramifiés ou à chaîne droite, substitué ou non substitué, qui comprend un ou plusieurs atomes de fluor ; et $R^2$ et $R^3$ sont indépendamment des groupes d'hydrocarbures ramifiés ou à chaîne droite, substitués ou non substitués.

**35.** Récipient selon la revendication 34, dans lequel les groupes d'hydrocarbures comprenant du fluor comprennent des groupes $R^1$ tels que définis dans l'une quelconque des revendications 13 à 15 ou 21.

**36.** Récipient selon l'une des revendications 34 ou 35, lequel récipient est composé d'un métal et/ou d'un plastique ou d'un matériau polymère.

**37.** Récipient selon la revendication 36, lequel récipient est composé d'aluminium, d'acier inoxydable, d'une résine de polyformaldéhyde et/ou d'un téréphtalate de polybutylène.

**38.** Système d'administration d'un médicament, qui comprend un récipient avec une surface interne modifiée tel que défini dans l'une quelconque des revendications 34 à 37.

**39.** Système selon la revendication 38, qui comprend un aérosol-doseur ou un aérosol-doseur pressurisé pour l'administration d'un médicament pulmonaire ou nasal.

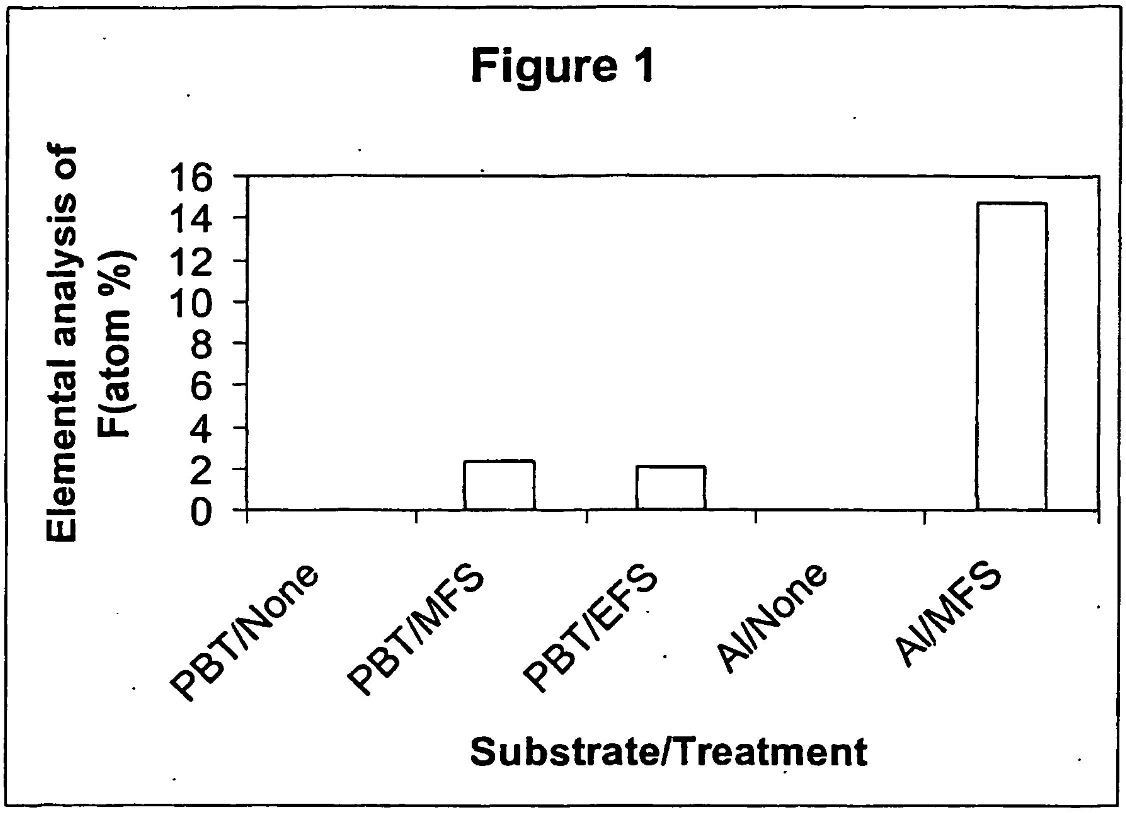
Figure 1
Elemental analysis of F(atom %)
16
14
12
10
8
6
4
2
0
PBT/None
PBT/MFS
PBT/EFS
Al/None
Al/MFS
Substrate/Treatment

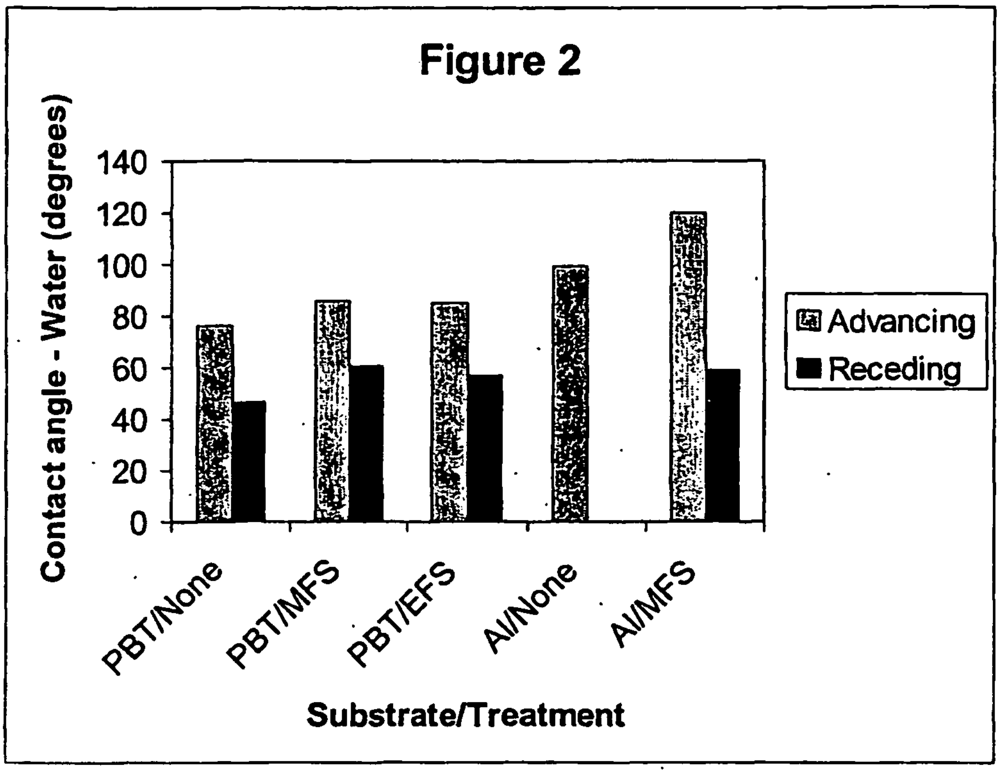
Figure 2
Contact angle - Water (degrees)
140
120
100
80
60
40
20
0
PBT/None
PBT/MFS
PBT/EFS
Al/None
Al/MFS
Substrate/Treatment
Advancing
Receding

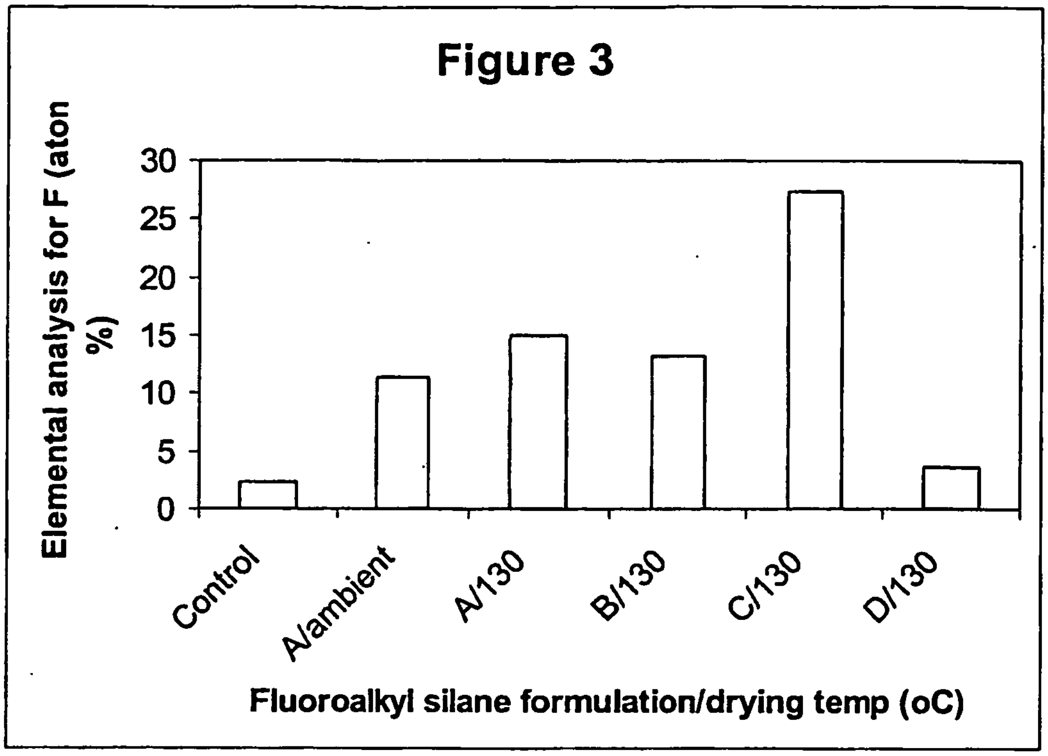
Figure 3
Elemental analysis for F (aton %)
30
25
20
15
10
5
0
Control
A/ambient
A/130
B/130
C/130
D/130
Fluoroalkyl silane formulation/drying temp (oC)

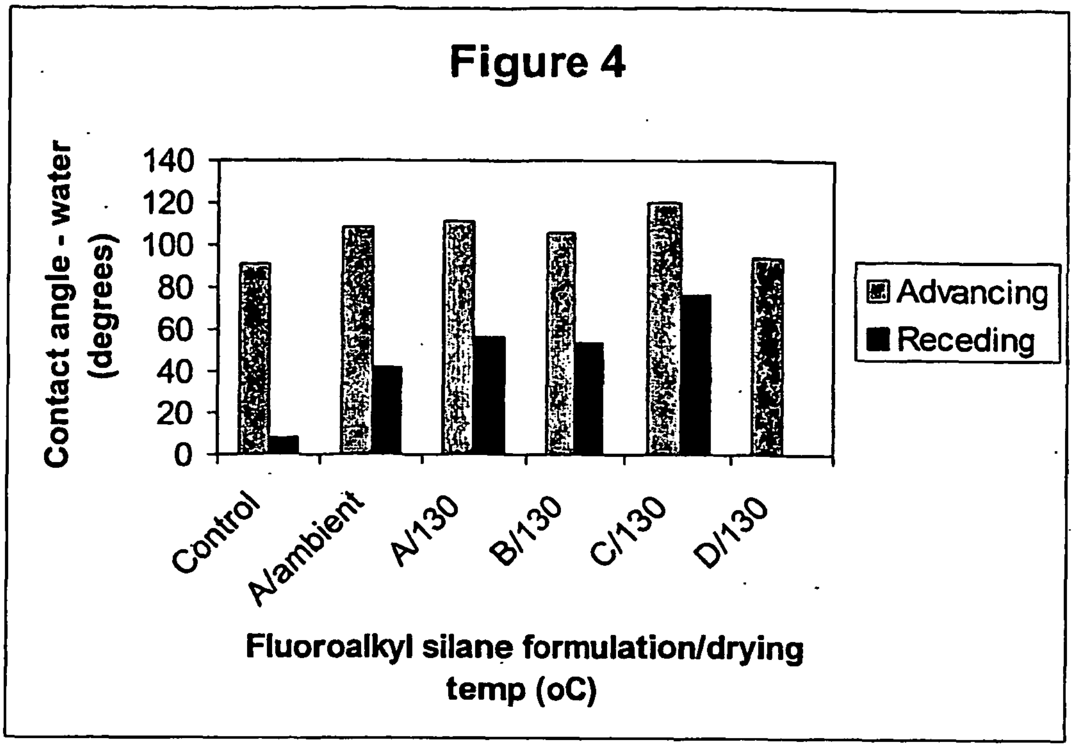
Figure 4
Contact angle - water (degrees)
140
120
100
80
60
40
20
0
Control
A/ambient
A/130
B/130
C/130
D/130
Advancing
Receding
Fluoroalkyl silane formulation/drying temp (oC)

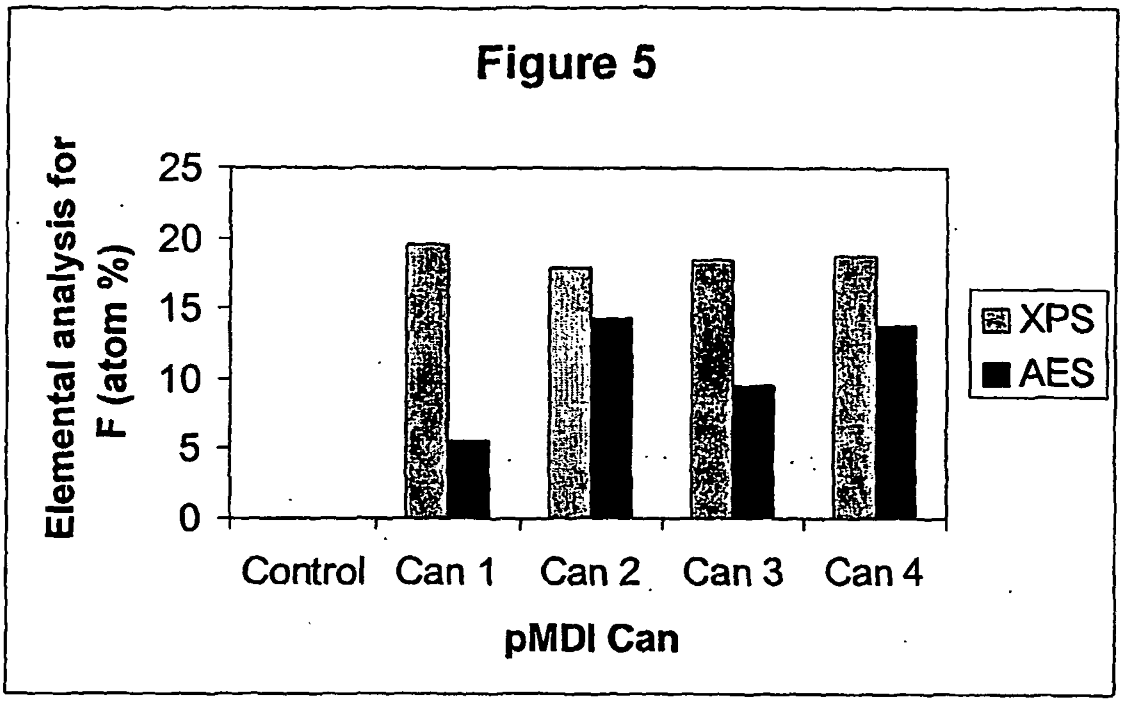
Figure 5
Elemental analysis for F (atom %)
25
20
15
10
5
0
Control
Can 1
Can 2
Can 3
Can 4
pMDI Can
XPS
AES

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0565743 A
- EP 1113064 A
- EP 0642992 A
- WO 9632150 A
- WO 9632345 A
- GB 2355252 A


### Non-patent literature cited in the description


- **KAWASE et al.** *J. Adhesion Sci. Technol.,* 1997, vol. 11 (11), 1381-1397
- **KHALADAR.** *Mat. Performance,* 1994, vol. 33 (2), 35-9
- **D.K. OWENS ; RC. WENDT.** *J. Appl. Polym. Sci.,* 1969, vol. 13, 1741
- **R.J. GOOD.** *J. Adhesion Sci. Terminol.,* 1992, vol. 6 (12), 1269-1302